Europäisches Patentamt

(19) European Patent Office

Office européen des brevets

(11) Publication number: **0 230 784**
A1

(12) # EUROPEAN PATENT APPLICATION

(21) Application number: 86310134.1

(22) Date of filing: 24.12.86

(51) Int. Cl.³: **C 12 N 15/00**
C 12 N 7/00, G 01 N 33/569
C 12 P 21/00
//A61K39/21

(30) Priority: 24.12.85 US 813069

(43) Date of publication of application:
05.08.87 Bulletin 87/32

(84) Designated Contracting States:
AT BE CH DE FR GB IT LI LU NL SE

(71) Applicant: THE UNITED STATES OF AMERICA as
represented by the Secretary U.S. Department of
Commerce
National Technical Information Service Office of
Government Inventions and Patents 5285 Port Royal
Road
Springfield, Virginia 22161(US)

(72) Inventor: Shaw, George Mead
3571 Rock Hill Road
Birmingham Alabama 35223(US)

(72) Inventor: Wong-Staal, Flossie
8418 Harker Drive
Potomac, Maryland 20854(US)

(72) Inventor: Fisher, Amanda Gay
5514 Johnson Avenue
Bethesda, Maryland 20817(US)

(72) Inventor: Gallo, Robert Charles
8513 Thornden Terrace
Bethesda, Maryland 20834(US)

(74) Representative: Daley, Michael John et al,
F.J. CLEVELAND & COMPANY 40/43 Chancery Lane
London, WC2A 1JQ(GB)

(54) plasmid and phage clones of human T-cell lymphotropic virus type III.

(57) Molecular clones of HTLV-III/LAV are disclosed. Clones λ
HXB-2, λ HXB-3, pHXB-2D, and pHXB-3D incorporate the full
length integrated proviral nucleotide sequences of HTLV-III/
LAV. The first two are inserted into λphage; the latter two
contain the viral sequences in a plasmid vector and are cytopathic for T-cell cultures and infectious *in vitro*.

EP 0 230 784 A1

./...

FIG 2

# PLASMID AND PHAGE CLONES OF HUMAN
# T-CELL LYMPHOTROPIC VIRUS TYPE III

Molecular clones of HTLV-III/LAV are disclosed. Clones λHXB-2, λ HXB-3, pHXB-2D, and pHXB-3D incorporate the full length integrated proviral nucleotide sequences of HTLV-III/LAV. The first two are inserted into λ phage; the latter two contain the viral sequences in a plasmid vector and are cytopathic for T-cell cultures and infectious in vitro.

Furthermore, the plasmid clones pHXB-2D and pHXB-3D may be used to generate a virus of a single genotype, therefore permitting the use of the plasmid clone to test the efficacy of new acquired immune deficiency syndrome (AIDS) drugs or vaccines.

## Background

Acquired immune deficiency syndrome (AIDS) is an epidemic immunosuppressive disease characteristically asso- ciated with a depletion of T-4 (helper) lymphocytes. A group of retroviruses, designated as human T-cell lympho- tropic virus Type III (HTLV-III), lymphadenopathy- associated virus (LAV), and AIDS-related virus (ARV), are variants of the same virus implicated in the pathogenesis of AIDS.

The molecular clones of the present invention contain integrated proviral DNA of HTLV-III. In the life cycle of a retrovirus, the single-stranded RNA genome must be copied by reverse transcriptase into an unintegrated linear DNA form prior to its integration into the host cell's chromosomal DNA. The linear unintegrated DNA duplex contains a copy of the viral genome in a nonpermuted order and can generally be detected only as a short-lived replication intermediate in the cytoplasm of infected cells shortly after viral infection. During integration, recombination of viral genetic elements with cellular genetic elements occurs; i.e., the cell is transformed and the viral genetic information is reproduced with the cells.

The unintegrated linear DNA form of H9/HTLV-III noted above has been cloned. These phase clones, desig-

nated λ BH-10, λBH-8, and λ BH-5, are described in Wong-Staal, et al., U.S. Patent Application Serial No. 643,306, filed August 22, 1984. The clones of integrated proviral DNA and unintegrated linear DNA are similar to each other but are distinguishable by differences in several cleavage sites. λ BH-10, λ BH-8, and λ BH-5 are incomplete viral clones that lack a short Sst I-Sst I segment of approximately 190 base pairs in the 5' LTR-leader sequence region. These sequences contribute to the ability to produce a pure virus population. Furthermore, the phage clones of the present invention, λ HXB-2 and λHXB-3, when inserted into a plasmid vector, are infectious _in vitro_, express viral antigen, produce mature virus particles, and exhibit marked cytopathic effects _in vitro_.

Analysis of the HTLV-III proviral clones of the present invention shows that the viral genome is approximately 10 Kb in length and possesses non-coding elements, LTR elements, at each end. Comparison of these full-length proviral clones of HTLV-III with clones of linear unintegrated viral DNA derived from the same HTLV-III-infected H9 cells (clones λ BH-10, λ BH-8, and λ BH-5) indicates that they are similar, but not identical, in restriction patern.

Description of the Figures

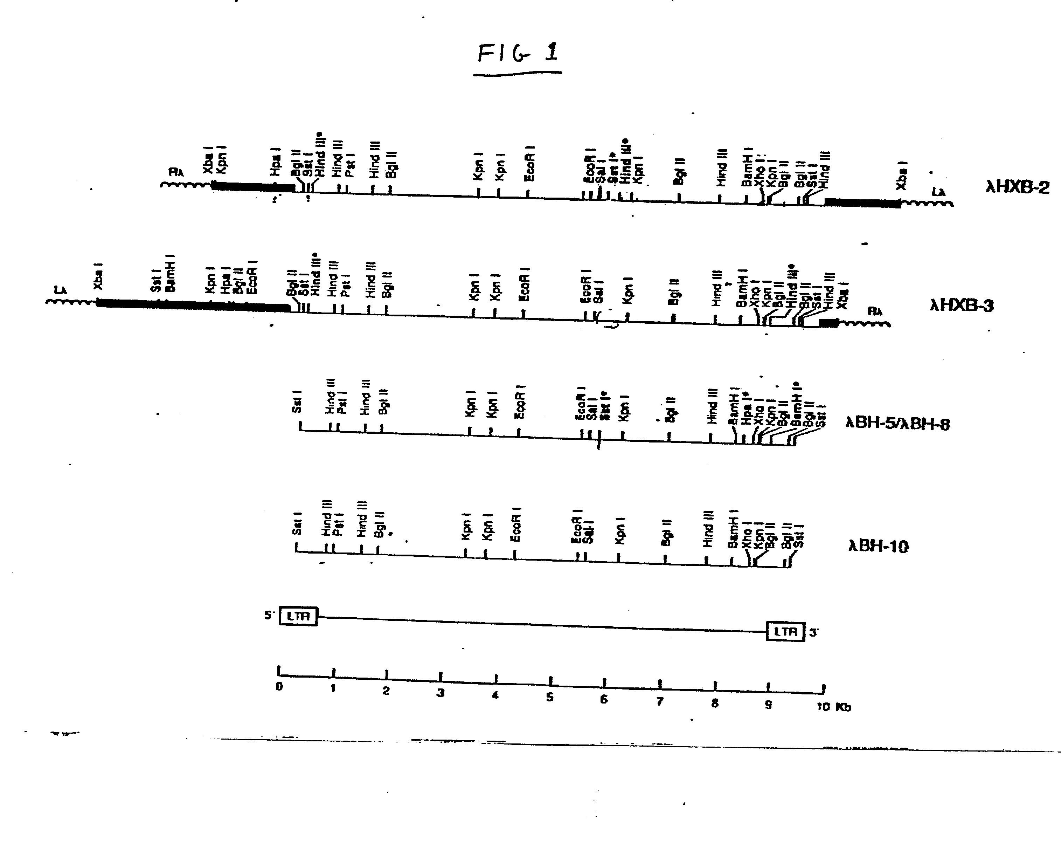

Figure 1 shows restriction endonuclease maps of four closely related clones of HTLV-III. λ HXB-2 and λHXB-3 represent full-length integrated proviral forms of HTLV-III obtained from the λ phase library of H9/HTLV-III DNA (Example 1). These clones contain the complete provirus (thin lines) including two LTR regions plus flanking cellular sequences (heavy lines). The LTR regions are known to contain the three restriction enzymes sites Bgl II, Sst I, and Hind III as shown, but their overall lengths are estimated. Clones λBH-10 and λ BH-5/λ BH-8 were derived from the linear unintegrated replicative intermediate form of HTLV-III in acutely infected H9 cells and have been reported in Hahn, et al., _Nature_, Vol. 312, pp. 166-169 (1984). Their restriction maps are shown here for

comparison with λHXB-2 and λHXB-3 and with Southern blots of genomic DNA from other HTLV-III containing cells. It should be noted that λBH-5/λBH-8 consists of two separately cloned Sst I fragments (λBH-5 and λBH-8) which together constitute one HTLV-III genomic equivalent but which are not necessarily derived from the same viral molecule. Also, because λBH-10 and λBH-5 were cloned with the restriction enzyme Sst I, they lack 5' LTR sequences as shown. Other differences in the restriction maps between these HTLV-III clones are indicated by bold letters and asterisks, with λBH-10 being used as a reference.

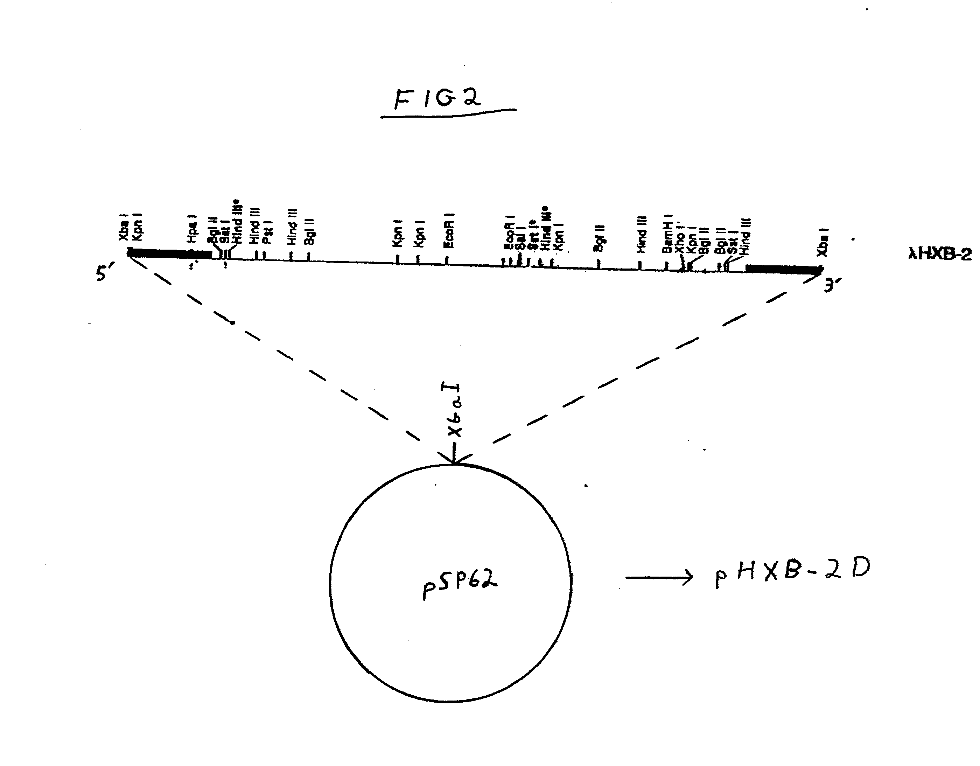

Figure 2 shows the construction of a plasmid containing HTLV-III. A 12.7-kb XbaI fragment derived from λHXB-2, a molecular clone containing about 10 kb of HTLV-III proviral sequences, was inserted into the XbaI site in the polylinker of plasmid pSP62 to produce the plasmid clone pHXB-2D. This plasmid construct was then transfected into DH-1 bacteria and used in protoplast fusion experiments. The thin horizontal line represents HTLV-III and the solid boxes represent flanking cellular sequences.

<u>Statement of Deposit</u>

Molecular clones λHXB-2, λHXB-3, pHXB-2D and pHXB-3 have been deposited in the American Type Culture Collection (ATCC, 12301 Parklawn Drive, Rockville, Maryland, 20852-1776 USA) under ATCC Nos. 40231, 40232, 67082 and 67081, respectively on May 2, 1986.

<u>Specific Disclosure</u>

The present invention may be represented in general terms by the following flow diagram:

Proviral HTLV-III DNA sequences

| λHXB-2 | Phage clone |
| pHXB-2D | Plasmid clone |
| H9/Clone | Immortalized cell producing HXB-2 virus |

Sequences of human T-cell lymphotropic virus type III (HTLV-III) were first detected in DNA of infected H9 cells (H9/HTLV-III) by Southern blot analysis with the use of a $^{32}$P-labeled complementary DNA (cDNA) probe prepared from HTLV-III virions. There was no evidence of such sequences in uninfected H9 cells. Preliminary analyses of Southern digests of H9/HTLV-III DNA revealed that the virus was present in this cell line both as unintegrated DNA and as proviral DNA integrated into the cellular genome at multiple different sites. Since the HTLV-III provirus was found to lack Xba I restriction sites, a genomic library was constructed by using Xba I-digested H9/HTLV-III DNA (cellular DNA), and this was screened with an HTLV-III cDNA probe to obtain molecular clones of full-length integrated provirus with flanking cellular sequences. Fourteen such clones were obtained from an enriched library of $10^6$ recombinant phage, and two of these were plaque-purified and characterized. Of these, phage clones $\lambda$HXB-2 and $\lambda$ HXB-3, which contain full-length integrated provirus (about 10 kilobases) with cellular flanking sequences (12.7 kilobases total length), were inserted into the Xba I site in the polylinker of plasmid pSP62 in order to produce plasmid clone pHXB-2D and pHXB-3D, respectively. This plasmid construct was then transfected into DH-1 bacteria for use in protoplast fusion experiments.

In the preferred protoplast fusion protocol, single colonies of bacteria bearing the plasmid are grown in L-broth containing ampicillin to a density of 2-4 x $10^8$ ml$^{-1}$ and chloramphenicol is added to a final concentration of 250 μg per ml of broth for plasmid amplification. After incubation at 37°C for 2 h, gentamycin is added to 10 μg ml$^{-1}$ and the cultures are re-incubated for 14-18 h to allow plasmid amplification. The bacteria are collected and resuspended in 2.5 ml of HEPES-buffered saline (HBS) pH 8.0, containing 20% sucrose. This suspension is placed on ice and at 5-min intervals, 0.8 ml of lysozyme solution (10 mg ml$^{-1}$ in 0.25 M Tris-HCl, pH 7.0), 1.0 ml of 0.25 M EDTA pH 8.0, and 1.0 ml of HBS are added sequentially. The suspension is incubated at 37°C for 10-30 min until 90% of the bacteria has formed protoplasts, then held on ice and slowly diluted to 50 ml with RPM1-1640 medium. For each

fusion, $10^{10}$ bacterial protoplasts are combined with 2.5 X $10^6$ PHA-stimulated core blood cells, washed and the pellet resuspended in 0.1 ml of RPMI-1640 medium. To these cells, 0.8 ml of polyethylene glycol fusion reagent (PEG-FR) is added dropwise over 1 min, the cells are left to stand for a further minute and then gradually diluted with RPMI-1640. PEG-FR is prepared as a 48% solution in RPMI-1640 medium and is pretreated by passing it over a resin bed as described in Yoakum, et al., _Science_, Vol. 22, pp. 385-389 (1983). After fusion, the cells are washed and returned to culture.

Also within the scope of this invention, an _in vitro_ model system to test the efficacy of drugs or vaccines potentially effective against AIDS has been established. The clones of the present invention represent the first proof that the HTLV-III genome alone is cytopathic (rather than a co-factor). These clones, therefore, are preferred in test protocols and test kits. In one such protocol, using pHXB-2D as an example, cord blood cells are treated with the test drug or vaccine and then exposed to pHXB-2D or H9/pHXB-2D. In another protocol, cord blood cells are first exposed to pHXB-2D or H9/pHXB-2D and then suspended in culture tubes containing the test drug or vaccine. The capacity of the drug or vaccine for treatment of AIDS can then be determined by measuring any block in infectivity or any block in the cytopathic effect of the virus.

The clones of the present invention may be transfected into a suitable cell line and the cells or antigen produced from those cells may be used as an immunogen for the production of antibodies which specifically bind to AIDS virus by bonding said clone to conventional immunogenic carrier material. The immunogen thus prepared is injected into a host using an adjuvant such as complete or incomplete Freund's adjuvant. The resulting anti-serum (obtained from the blood or spleen of the host) will contain antibodies which specifically bind to AIDS virus.

Conventional immunogenic carrier material is defined to include proteins and polypeptides. Suitable conventional proteins include but are not limited to human or bovine gamma globulin, or human, bovine, or rabbit serum albumin.

Test Kits

Clone pHXB-2D or pHXB-3D may be transfected into an immortalizing cell line such as H9, and the cells, antigens, or proteins produced from those cells may be incorporated as an antigen in test kits for the diagnosis of AIDS in human blood. Such a kit and diagnosis procedure include contacting clone pHXB-2D or pHXB-3D (bound to a solid support) with a test sample of human blood. Any antibodies present in the sample will bind to said clone. After removing the test sample and washing the solid support (to remove unbound anti-body), the presence of human antibodies to AIDS virus may be detected by probing the solid support with a reagent capable of selectively detecting AIDS virus antibodies.

Immortalized cell lines containing pHXB-2D (or pHXB-3D) or segments obtained from the clone may be used in an enzyme linked immunosorbent assay (ELISA). A diagnostic test kit for the detection of AIDS-specific antibodies comprises a compartmented enclosure containing a plurality of wells or plates (employing a sorbent or porous surface) which are coated prior to use with antigen or protein derived from cells transfected with the plasmid clone or DNA sequences obtained from the plasmid clone. The clone bound to the porous surface is then incubated with a test sample of human blood. After removal of the sample, known methods of conducting an ELISA assay are employed: coating the wells with normal goat serum and peroxidase-labeled goat anti-human immunoglobulin and a color change indicator (such as orthophenylene diamine and hydrogen peroxide in a phosphate citrate buffer).

Other forms of a test kit may also be constructed. For example, competition radioimmunoassay or Dot Blot assays include a compartmented container with cover, a sorbent or porous surface, such as a nitro-cellulose sheet, surfactants, pH modifiers, bovine serum albumin, human Fab, dilute normal goat serum, and $^{125}$I-labeled animal anti-human immunoglobulin.

Clone pHXB-2D (or pHXB-3D) may be incorporated in an _in vitro_ test kit for the screening of new drugs or vaccines potentially effective against AIDS. This test kit contains a source of virus (pHXB-2D or H9/pHXB-2D), cord blood cells, and polybrene. Cord blood cells are exposed to various concentrations of the test drug, polybrene, the cells are pelleted, and then exposed to pHXB-2D or H9/pHXB-2D. The cells are then suspended in fresh complete medium and cultured for several days. One of several means of detecting infectivity or cytopathic effect is then used to determine the capacity of the test drug against AIDS.

As has been mentioned above, the virus that causes AIDS exists in many forms - HTLV-III, LAV, and ARC, to name a few. It has been shown that the restriction patterns of each of these strains, as well as isolates within each strain, vary slightly but are essentially the same virus. It is not intended that the scope of this invention be limited by these slight differences. Accordingly, wherever the term HTLV-III or Human T-Cell lymphotropic virus type III is used, it should be understood to include all forms of the virus that causes AIDS.

Example 1

A λ phage library was constructed according to standard methods (Maniatis, et al., _Molecular Cloning - A Laboratory Manual_, 1982), using the cloning vector J1 λ and Xba I digested H9/HTLV-III DNA which had been enriched by sucrose gradient centrifugation for 10- to 15-kb fragments. Phage plaques ($10^6$) were screened with cDNA prepared from doubly banded HTLV-III virions and 14 positive signals were obtained. Two of these were plaque purified and their restriction maps determined, as shown in Figure 1 (λ HXB-2 and λHXB-3).

Example 2

To determine whether the HTLV-III genome contains sequences homologous to normal human DNA, the viral insert of λ HXB-2 (5.5 kb and 3.5 kb Sst I-Sst I fragments) was isolated, nick translated, and used to probe HTLV-III-infected and uninfected cellular DNA. Under standard conditions of hybridization [washing conditions: 1 x SSC (standard saline citrate), 65°C; annealing temperature ($T_m$), -27°C], this probe hybridized to DNA from H9/HTLV-III cells as well as other HTLV-III-infected cells, but not to DNA from uninfected H9 cells, uninfected HT cells (the parent cell line from which H9 is cloned), or normal human tissues. This finding is in agreement with the results of other experiments in which the unintegrated (replicative intermediate) form of HTLV-III was used as probe and demonstrates that HTLV-III, like HTLV-I and HTLV-II, is an exogenous retrovirus lacking nucleic acid sequences derived from human DNA.

Example 3

Kinetics of cell growth and reverse transcriptase activity in cord blood mononuclear cell cultures following protoplast fusion. Mononuclear cells were prepared from cord blood samples using Ficoll Triosil and cultured for 5 days in media containing PHA. These cells were then fused with bacterial protoplasts carrying the plasmid pHXB-2D, pSV2neo or pCH-1gpt and maintained in culture at a density of 5 x $10^5$ cells $ml^{-1}$ by addition of RPMI-1640 medium containing 20% fetal calf serum, 10% T-cell growth factor (inter-leukin-2) and antibiotics. Three parallel fusions using cells from different individuals were established for each plasmid. Spent medium removed from two cultures at 5, 11, 14 and 18 days after fusion was concentrated 10-fold and assayed for the presence of reverse transcriptase using standard techniques. The activity detected in each of the culture supernatants is expressed as the amount of $^3$H-labeled deoxyribonucleoside monophosphate ($^3$H-dTMP) incorporated (in pmol per 0.3-ml sample) using $dT_{15}\cdot(rA)_n$ as the template primer.

The growth of all cultures was comparable for the first 14 days after protoplast fusion. By day 18, however, the number of viable cells in cultures transfected with pHXB-2D had fallen dramatically: there was a 10-fold and a 100-fold reduction between days 18 and 21 and 18 and 32, respectively. Cultures transfected with either pSV2neo or pCH-1gpt showed only a 4-5-fold reduction over the same time period. When supernatant from the cultures was assayed for the presence of reverse transcriptase, activity was detected exclusively in cultures transfected with pHXB-2D. These data suggest that replicating virus was present in cultures 11-18 days after fusion with pHXB-2D protoplasts.

Example 4

Expression of the HTLV-III gag-related proteins p15 and p24 by transfected cells was demonstrated using specific monoclonal antibodies: maximum expression was observed 18 days after transfection, when 4-11% and 5-9% of cells were reactive with antibody to p15 and p24, respectively. Virus particles were detected by electron microscopy in all cultures 14-18 days after transfection with pHXB-2D. The particles contained condensed, truncated cores, which are characteristic of HTLV-III particles.

Example 5

In time-course experiments, DNA isolated from a single culture 6, 11, 14, 18 and 31 days after transfection with pHXB-2D, was digested with BamHI and analyzed for HTLV-III sequences. Six days after transfection, an 8.6-kb DNA fragment was detected as a faint band; 18 days after transfection it was possible to detect a 1.5-kb DNA fragment in addition to the 8.6-kb fragment. The total amount of unintegrated virus in the cultures appeared to increase, as suggested by the increase in intensity of these bands with time; this is evidence that cells originally transfected with pHXB-2D are able to produce fully infectious virus which is then transmitted within the culture.

No HTLV-III sequences were detected 31 days after transfection; at this point the culture may have contained only cells which failed to be infected by HTLV-III. This result is again consistent with the transfected DNA exerting a cytopathic effect on T cells. The finding that, at any stage, only a minor population of the transfected cells are apparently infected by the virus (< 15% express viral proteins) suggests that the cytopathic effects may not result solely from direct viral infection and that secreted factors and/or other cell-to-cell interactions may play a part in the cytopathic phenomenon.

4.    A molecular clone substantially characterised by
the following restriction endonuclease map:

λHXB-2

## Claims

1. A biologically competent recombinant clone of HTLV-III containing integrated human T-cell lymphotropic virus type III proviral DNA sequences and being infectious in vitro and cytopathic for T-cell cultures.

2. A molecular clone of human T-cell lymphotropic virus type III characterised by comprising a phage clone, containing a 12.7 kilobase Xba I fragment, said fragment comprising a 10 kilobase full length integrated provirus and 2.7 kilobase cellular flanking sequences.

3. A molecular clone of human T-cell lymphotropic virus type III, characterised by comprising a clone containing integrated human T-cell lymphotropic virus type III proviral DNA sequences, said clone expressing viral antigen and producing mature virus particles.

5. A molecular clone of human T-cell lymphotropic virus type III comprising the molecular clone designated λ HXB-2.

6. A process for the production of a molecular clone of a virus wherein a genomic library is constructed using cellular DNA and where the clone is separated from the library using a viral cDNA probe, characterised by producing a molecular clone of human T-cell lymphotropic virus type III, said clone containing full length integrated provirus nucleotide sequences.

7. A process as claimed in claim 5 characterised by constructing a phage library using a suitable cloning vector and endonuclease digested human T-cell lymphotropic virus type III, enriching said library for 10-15 kilobase DNA fragments by sucrose gradient centrifugation, and screening said library using a cDNA probe prepared from doubly banded human T-cell lymphotropic virus type III virions, and obtaining a phage clone containing full-length integrated proviral nucleotide sequences of human T-cell lymphotropic virus type III.

8. The process of claim 6 or 7, further characterised in that the provial sequences are the XBa I fragment of human T-cell lymphotropic virus type III.

9. A test plate or sheet for use in an immunoassay, characterized in that it includes thereon antigen or protein derived from cells transfected with human T-cell lymphotropic virus type III molecular clone pHXB-2D.

10. A test kit for use in the diagnosis of acquired immune deficiency syndrome, characterized in that it comprises a sorbent or porous surface including thereon antigen or protein derived from cells transfected with human T-cell lymphotropic virus type III molecular clone pHXB-2D.

11. A process for the production of antibodies which specifically bind to a human retrovirus which causes acquired immune deficiency syndrome, characterized in that it comprises bonding molecular clone pHXB-2D or H9/pHXB-2D to an immunogenic carrier to form an immunogen, injecting said immunogen into a suitable host, and obtaining antibodies from the blood or spleen of said host, said antibodies specifically binding to the virus which causes acquired immune deficiency syndrome.

12. A test kit used for the diagnosis of acquired immune deficiency syndrome wherein an antigen is bound to a sorbent or porous substrate, characterized in that said antigen contains part or all of the DNA sequence of clone pHXB-2D.

13. A test kit characterized in that it is useful for analyzing the capacity of a drug or vaccine against acquired immune deficiency syndrome and comprises cord blood cells, a source of pHXB-2D, culture medium, and polybrene.

14. Molecular clone λHXB-3 substantially characterised by the following restriction endonuclease map.

```
           ⌐
         ╫ Xba I

         ┤ Sst I
           BamH I

         ┤ Kpn I
           Hpa I
           Bgl II
           EcoR I

         ┤ Bgl II
         ═ Sst I
           Hind III*
         ┤ Hind III
         ┬ Pst I

         ┤ Hind III
         ┤ Bgl II



         ┤ Kpn I

         ┤ Kpn I

         ┤ EcoR I


         ┤ EcoR I
         ⸗ Sal I

         ┤ Kpn I


         ┤ Bgl II


         ┤ Hind III

         ┤ BamH I
           Xho I
           Kpn I
           Bgl II
           Hind III*
           Bgl II
           Sst I
           Hind III
           Xba I
           ⌐
```

λHXB-3

15.  A molecular clone characterised by comprising any one of λHBX-2, λHBX-3, PHXB-2D and pHXB-3D as deposited in the ATCC under Nos. 40231, 40232 67082 and 67081.

FIG 1

λHXB-2

λHXB-3

λBH-5/λBH-8

λBH-10

5' LTR          LTR 3'

0   1   2   3   4   5   6   7   8   9   10 Kb

FIG 2

λHXB-2

pSP62

→ pHXB-2D

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | SCIENCE, vol. 226, 7th December 1984, pages 1165-1171; G.M. SHAW et al.: "Molecular characterization of human T-cell leukemia (lymphotropic) virus type III in the acquired immune deficiency syndrome" <br> * Whole article, especially figure 1 * | 1-8,14 ,15 | C 12 N 15/00 <br> C 12 N 7/00 <br> G 01 N 33/569 <br> C 12 P 21/00 // <br> A 61 K 39/21 |
| X | NATURE, vol. 316, no. 6025, 18th July 1985, pages 262-265, London, GB; A.G. FISHER et al.: "A molecular clone of HTLV-III with biological activity" <br> * Whole article * | 1-8,15 | |
| X | CELL, vol. 40, January 1985, pages 9-17, MIT; S. WAIN-HOBSON et al.: "Nucleotide sequence of the AIDS virus, LAV" <br> * Page 9, column 2 * | 1,6,7 | TECHNICAL FIELDS SEARCHED (Int. Cl 4) <br><br> C 12 N |
| X,D | NATURE, vol. 312, no. 5990, 8th November 1984, pages 166-169, Reading, Berks, GB; B.H. HAHN et al.: "Molecular cloning and characterization of the HTLV-III virus associated with AIDS" <br> * Figure 2 * | 1 | |

The present search report has been drawn up for all claims

| Place of search | Date of completion of the search | Examiner |
|---|---|---|
| THE HAGUE | 01-04-1987 | CUPIDO M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82